# EUROPEAN PATENT SPECIFICATION

(11) **EP 0 326 757 B1**
(45) Date of publication and mention of the grant of the patent: **21.07.1993**
(21) Application number: 88312049.5
(22) Date of filing: 20.12.1988
(51) Int. Cl.: A61B 17/11

(54) **Apparatus for anastomosing digestive tract**
Vorrichtung zur Anastomose des Verdauungssystems
Dispositif pour effectuer une anastomose du tube digestif

(30) Priority: 05.02.1988 JP 24996/88
(43) Date of publication of application: 09.08.1989
(73) Proprietor: Fujitsuka, Tatsuo, Minamisaitama-gun Saitama (JP); Kawasaki, Hiroshi, Tokyo (JP)
(72) Inventor: Fujitsuka, Tatsuo, Minamisaitama-gun Saitama (JP); Kawasaki, Hiroshi, Tokyo (JP)
(74) Representative: Sheader, Brian N.

(56) References cited:
- EP-A- 0 119 848
- US-A- 4 638 056
- US-A- 4 708 141

## Description

The present invention relates to an apparatus for anastomosing cut ends of a digestive tract extending from the esophagus to the rectum of the anus thereabout.

One of known apparatuses for anastomosing cut ends of a severed digestive tract during a surgical operation of the digestive tract is disclosed in Japanese Utility Model Publication No. 46-16477.

The disclosed apparatus includes a pair of ring-like joining members supported onto a pipe and brought into pressure contact with each other by a spring of magnetic force, with both cut ends to be anastomosed being held between the pair of joining members. After adhesion of the cut ends, the members used are removed through the esophagus or excreted through the rectum.

The foregoing conventional apparatus is effective when used in the esophagus, the rectum and so on from which it can easily be discharged or excreted. When it is used in the remaining portions, there arises a problem that a relatively large-sized solid foreign matters must be discharged or excreted in the same size as they have been set. Another problem is in that the conventional apparatus can be applied to end-to-end anastomosis as shown in Fig. 15 and end-to-side anastomosis as shown in Fig. 16, but it is not applicable to side-to-side anastomosis as shown in Fig. 17.

US 4 708 141 discloses a soluble suturing device which has the features of the pre-characterising clause of claim 1. This device requires the use of staples to hold the severed ends of the digestive tract.

The present invention has been accomplished in view of the foregoing status in the art. It is an object of the present invention to provide an apparatus for anastomosing a digestive tract which requires the excretion of only small-sized foreign matters after adhesion of cut ends, thereby ensuring safety and lessening a burden imposed on patients.

The apparatus for anastomosing a digestive tract of the present invention is featured in that a support tube is formed of mainly a soluble material, an annular groove is defined in the outer periphery of the support tube, and a plurality of retainers each endowed with a property of closing its open both ends, disposed around the groove in the support tube at a plurality of locations along both the inner side surfaces and the inner bottom surface of the groove in a radial pattern as a whole, in a state that said both opposite ends are opened, the retainers being released from the support tube with their open opposite ends closed as the soluble material of the support tube is dissolved.

The soluble material of the support tube can be formed of saccharides. The retainers can be each formed in a substantially U-shaped or arcuate with said both ends thereof opened when they are embedded in the support tube, formed to have both ends opposite or overlapped with each other when said both ends are closed, or made of a shape memory alloy or resin endowed with a property of closing its open opposite ends at a predetermined temperature when used, or made using a spring member, or provided at its open opposite ends with magnets attracting to each other.

When using the apparatus of the present invention, opposite ends of the support tube are inserted into cut ends of a digestive tract to be anastomosed, respectively, and the cut ends are both engaged in the groove of the support tube. In this condition, as the soluble material of the support tube is dissolved, open opposite ends of each of a plurality of retainers are closed to hold by clipping or squeezing by the retainers the cut ends of the digestive tract in the same manner as the inner suture of the standard two-layer inversion anastomosis. Being left in that state, the cut ends of the digestive tract adhere to each other outside a plurality of retainers. When the parts of the anastomosed digestive tract held by the retainers inside the adhered portion are necrotized, the retainers fall off inside the digestive tract.

With the apparatus of the present invention, where saccharides are used as the soluble material of the support tube, human bodies are less affected. Further, where the retainers are each made of a shape memory alloy or resin, the force of closing the open opposite ends of each retainer can be sustained until the time of use, even when a storage period prior to use is prolonged, and the retainers can be easily buried into the support tube. Where the retainers using spring members, they can be manufactured less expensively. Where the retainers are each provided at its open opposite ends with magnets attracting to each other, holding portions of the cut ends of the digestive tract are brought into surface-to-surface pressure contact. Where the retainers are each formed in a circle when said both ends are closed, the retainers become suitable for mass production.

Other objects and features of the present invention will be described with reference to the drawings.

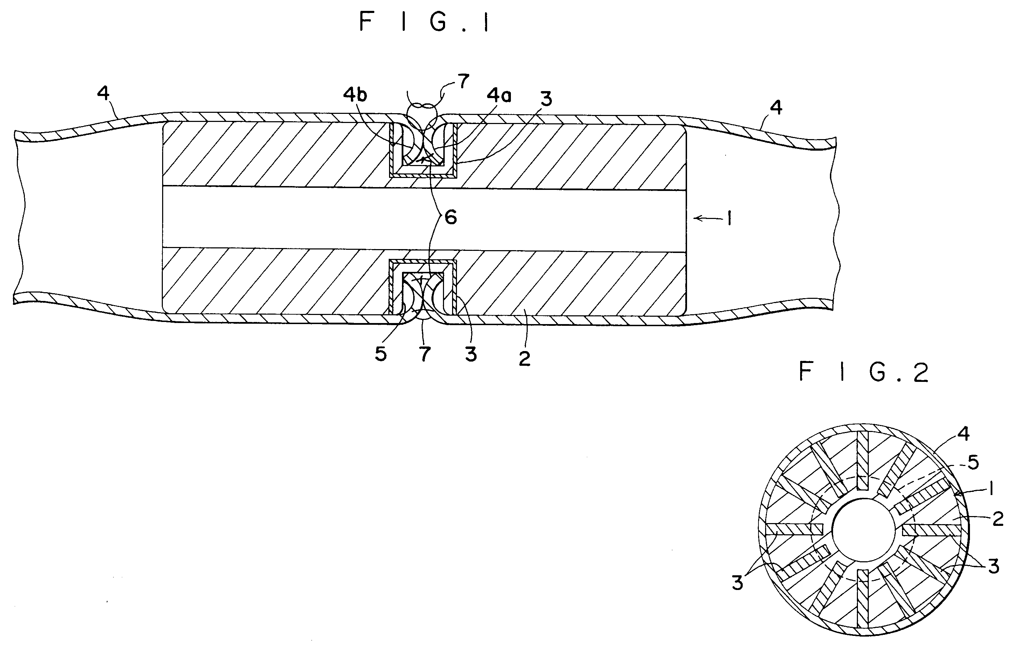

Figs. 1 and 2 are a longitudinal sectional view and a transverse sectional view showing one embodiment of an apparatus of the present invention when used, respectively;
Fig. 3 is a perspective view showing the state of a digestive tract prior to use of the anastomosing apparatus;

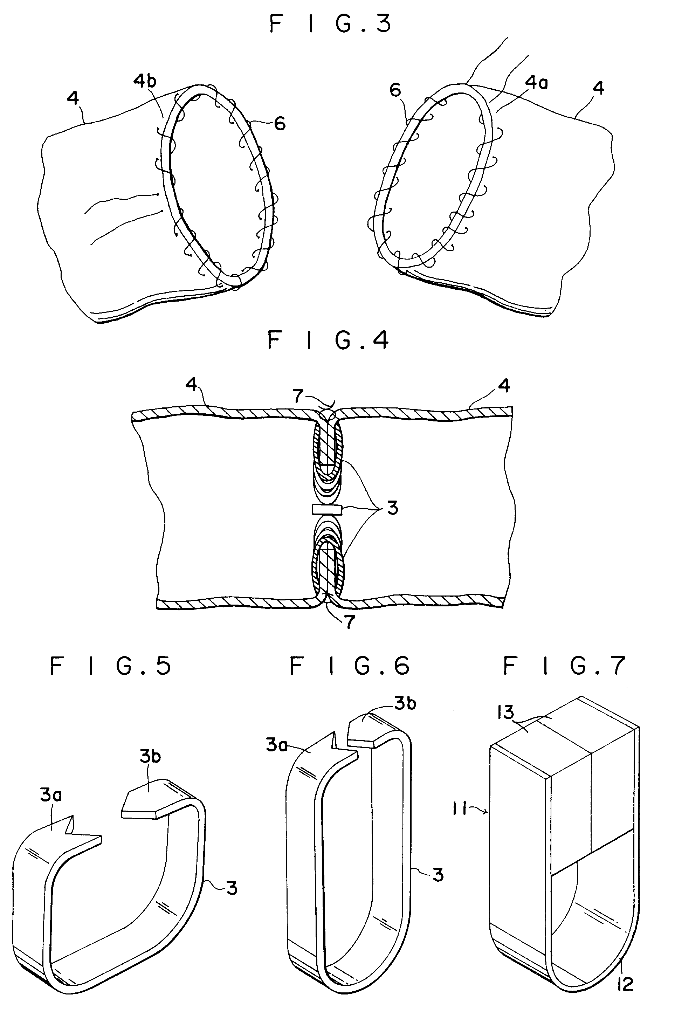

Fig. 4 is a longitudinal sectional view showing the state of the digestive tract after use of the anastomosing apparatus;
Figs. 5 and 6 are perspective views showing modifications of a retainer;
Fig. 7 is a perspective view showing another modification of the retainer;

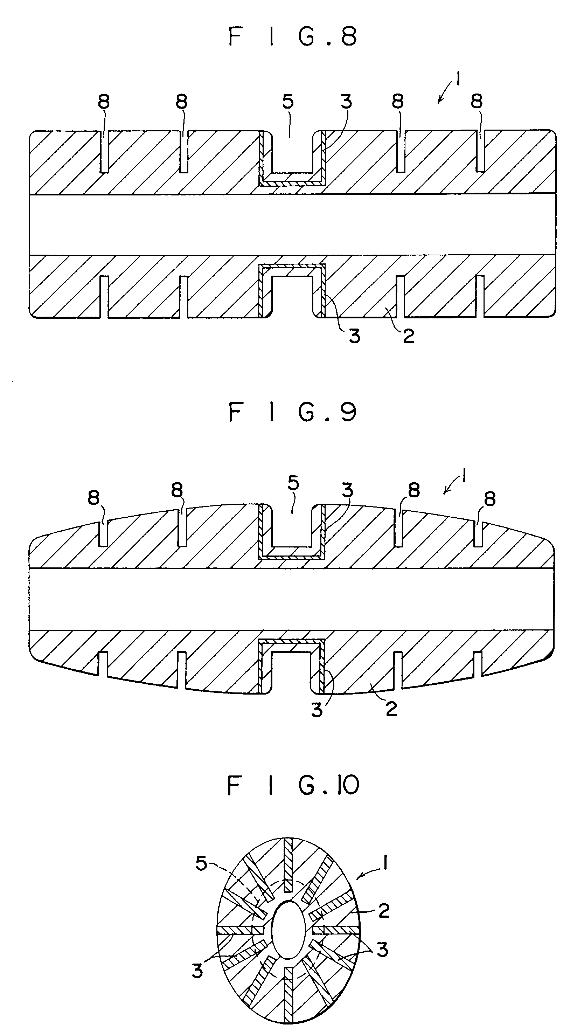

Figs. 8 through 10 are sectional views each showing a modification of a support tube;

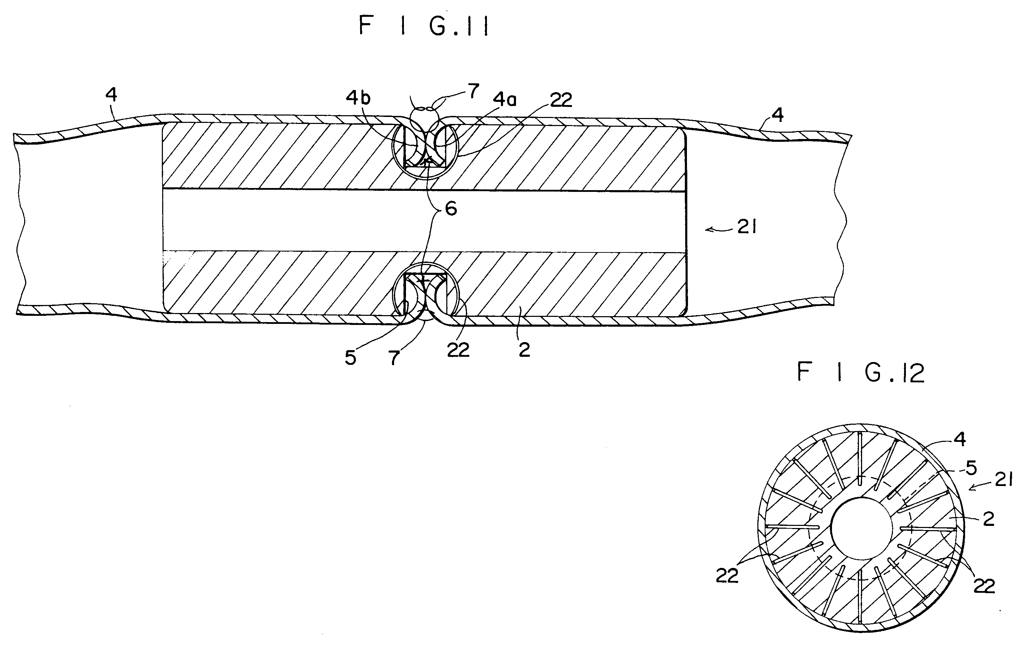

Figs 11 and 12 are a longitudinal sectional view and a transverse sectional view showing an other embodiment of an apparatus of the present invention when used, respectively;

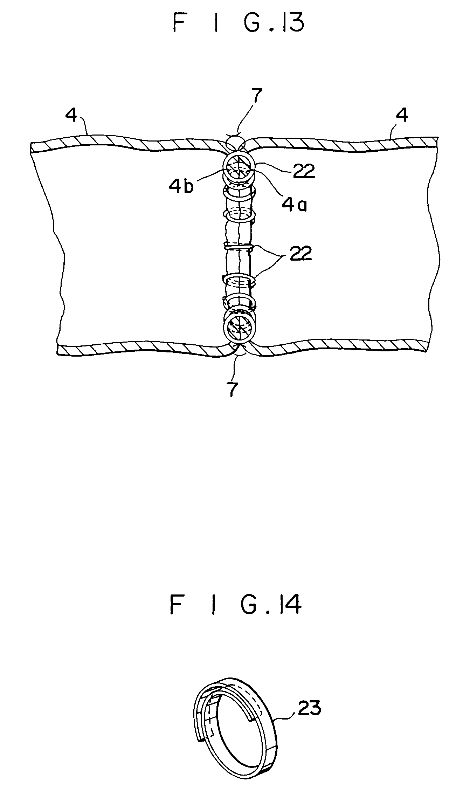

Fig. 13 is a longitudinal sectional view showing the state of the digestive tract after use;
Fig. 14 is a perspective view showing other modification of the retainer; and

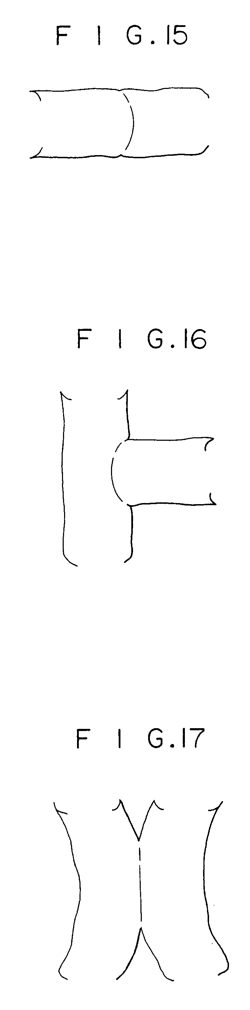

Figs. 15 through 17 are explanatory views of an anastomosing portion.

Preferred embodiments of the apparatus of the present invention will be described below with reference to the drawings.

In Figs. 1 and 2, designated at the reference numeral 1 is an anastomosing apparatus which is composed of a support tube 2 formed of mainly saccharides, e.g., disaccharides such as palatinose, as a soluble material, and a plurality of belt-like retainers 3 each formed of a shape memory alloy.

As an example case of end-to-end anastomosis both cut ends 4a, 4b of a digestive tract 4 severed, the support tube 2 has a cylindrical shape with outer diameter of 20 - 30 mm, inner diameter of 6 mm and length of 50 mm or thereabout, and a groove 5 is formed on the outer periphery in an annular shape with width of 4 mm and depth of 4 mm or thereabout. The belt-like retainers 3 have each an approximately 16 mm length and is shaped into a substantially U-form (channel-like form in the illustrated example). A total of 12 to 20 retainers 3 are arranged around the groove 5 of the support tube 2 in a radial pattern as a whole, with respective open opposite ends directed outward. Each retainer 3 is buried in the support tube 2 along both the inner side surfaces and the inner bottom surface of the groove 5 to a depth of approximately 1 mm therefrom.

Furthermore, the retainer 3 is buried in the support tube 2 in a channel-like configuration which has a spacing between its open opposite ends wider than that of the memorized intrinsic shape. The retainer 3 has such a property that it returns to the memorized intrinsic shape which has a spacing between its open opposite ends narrower than that in the configuration of Fig. 1, when the temperature of the retainer 3 is raised up to the body temperature of 36 to 40°C.

The apparatus 1 is used as follows. After excising the diseased portion of the digestive tract 4, both cut ends 4a, 4b of the digestive tract 4 are respectively tied up by running stitching using a thread 6 such as a non-processed (plain) cut gut which will be absorbed in about a week, as shown in Fig. 3. Then, as shown in Fig. 1, opposite ends of the support tube 2 are inserted into the cut ends 4a, 4b, respectively, and the cut ends 4a, 4b are engaged with the groove 5 of the support tube 2 by tightening the thread 6 used for the above running stitching. In this state, the outer layers of both the cut ends 4a, 4b are sutured with a thread 7 as many as 8 - 10 stitches for reinforcement.

With the lapse of time in that state, the support tube 2 falls into pieces gradually as the saccharides composing the support tube 2 is dissolved. After about 1 hour, a plurality of retainers 3 become separated from the support tube 2 as shown in Fig. 4. At the same time, since the temperature of the respective retainers 3 is raised by the body temperature, they are caused to return to their intrinsic shape, thereby closing open opposite ends. Thus, the cut ends 4a, 4b of the anastomosed digestive tract 4 are pinched and held by the retainers 3 in the same manner as the albert's suture of the standard two-layer inversion anastomosis.

Then, as time goes by in the above state, the cut ends 4a, 4b of the digestive tract 4 adhere to each other outside the radially arranged retainers 3. After one or two weeks, when the parts of the anastomosed digestive tract 4 held by the retainers 3 inside the adhered portion are necrotized, the retainers 3 fall off inside the digestive tract 4 and then automatically excreted.

As described above, since a plurality of retainers 3 used in the apparatus 1 of the present invention separately fall off after completing their intended mission and are each sufficiently small in size, the respective retainers 3 are automatically excreted without the need of special attention, with the result that safety is improved and a burden imposed on patients is lessened. The apparatus 1 of the present invention can be applied to any of the foregoing embodiments, end-to-end anastomosis as shown in Fig. 15, end-to-side anastomosis as shown in Fig. 16, as well as side-to-side anastomosis as shown in Fig. 17.

Further, since a plurality of retainers 3 are arranged in a radial pattern, the cut ends 4a, 4b can be held in the same manner as the standard two-layer inversion anastomosis and, blood stream in the region of the cut ends 4a, 4b is secured. In addition, the retainers 3 are each caused to close the open opposite ends with its own property, so it is not necessary to perform closing operation by the use of a special closure appliance, such as staples, which have been used in the past. Accordingly, there is no fear that the appliance may traumatize the organs and tissues in the surroundings, and there is no need of repairing the hole through which the appliance has been inserted. As a result, the possibility of stenosis is small, the post-operation recuperation becomes more satisfactory, and earlier recovery can be expected.

In the foregoing embodiment, for example, it may be modified such that engagement portions 3a, 3b are respectively formed at the open opposite ends of the retainer 3 as shown in Fig. 5, and the engagement portions 3a, 3b are caused to engage with each other as shown in Fig. 6, when the retainer 3 closes the open opposite ends. In addition to a shape memory alloy, the retainer 3 may be formed of a shape memory resin which has a similar property as the shape memory alloy, or a spring member. As alternative retainer 11 may be fabricated, as shown in Fig. 7, by attaching a pair of magnets 13 attracting to each other to open opposite ends of a connecting plate 12 made of synthetic resin or the like.

While the support tube 2 is mainly formed of a soluble material such as saccharides, it is not always necessary for the support tube 2 to be entirely soluble. For example, it may be fabricated by solidifying particles of an insoluble material, which will not affect human bodies, with soluble material. In addition to saccharides, starch bound with excipient, glue, and soluble synthetic resin may be included in soluble material.

Furthermore, with annular cutouts 8 formed on the outer periphery of the support tube 2, as shown in Figs. 8 and 9, the support tube 2 can be adjusted in its length by cutting it at any of the cutouts 8. This makes it possible to easily apply the apparatus 1 of the present invention to the end-to-side and side-to-side anastomoses as shown in Figs. 16 and 17 too. As an alternative, when the support tube 2 is shaped to have an ellipsoidal cross section as shown in Fig. 10, it becomes optimum to the side-to-side anastomosis as shown in Fig. 17. Though not shown, when the support tube 2 is bent to a substantially V-shape, it is suitable for use in the bent portion.

Figs. 11 through 13 show an anastomosing apparatus 21 of an another embodiment of the present invention. This embodiment is similar to the foregoing embodiment except the retainer and accordingly in the explanation of an anastomosing apparatus 21 of this embodiment, the same portions as those of the anastomosing apparatus 1 in the forgoing embodiment are designated by the same reference numerals and the detailed explanations thereof are omitted.

The retainer 22 in this embodiment is made of a shape memory alloy of a wire shape with length of about 13.3 mm and outer diameter of about 0.3 mm and returns to the memorized intrinsic shape when the temperature of the retainer 22 is raised up to about (30±5)°C. The groove 5 of the support tube 2 has width of 4 mm and depth of 4 mm or thereabout. The memorized intrinsic shape of the retainer 22 is a coil shape with diameter of about 2 or 3 mm, rounded about 2.1∼1.4 times, and at least both ends of which are overlapped. As shown in Figs. 11 and 12, a total of 15 to 24 retainers 22 are arranged around the groove 5 in the support tube 2 in a radial pattern as a whole spacing of about 4 mm, respectively, on the outer-periphery of the support tube 2 having outer diameter of about 20 to 30 mm. Each retainer 22 is buried in the support tube 2 along both the inner side surfaces and the inner bottom surface of the groove 5. Further, in the support tube 2 the retainer 22 is widened forcibly into a channel-like configuration circumscribing the square groove 5 of the support tube 2 having width of 4 mm and depth of 4 mm or thereabout, which has a spacing between its open opposite ends directed outward.

The apparatus 21 is used similar to the apparatus 1 mentioned already. After both cut ends 4a, 4b of the digestive tract 4 are processed as explained with reference to Fig. 3, the cut ends 4a, 4b of the digestive tract 4 are engaged with the groove 5 of the support tube 2 as shown in Fig. 11 and as explained with reference to Fig. 1.

With the lapse of time in that state, the support tube 2 is dissolved. At the same time, since the temperature of the respective retainers 22 is raised by the body temperature, they are caused to return to their intrinsic coil shape as shown in Fig. 13, so that the open opposite ends are penetrated into the cut ends 4a, 4b are closed. Thus, the cut ends 4a, 4b of the anastomosed digestive tract 4 are squeezed and held by the retainers 22 in the same manner as the albert's suture of the standard two-layer inversion anastomosis.

In this case, the cut ends 4a, 4b of the digestive tract 4 are squeezed under the shrinking state, because the cross-sectional area of the cut ends 4a, 4b of the digestive tract 4 squeezed by the retainers 22 are larger than the area of a circle having diameter of about 2 to 3 mm, which is the intrinsic shape of the retainer 22.

Specifically, if the running stitch by a thread 6 is formed on the digestive tract 4 at a portion apart by 2 mm from the cut ends 4a, 4b thereof, it is considered that the distance of 2 mm is reduced to one half, i.e., 1 mm when the thread 6 used for the running stitching is tightened to engage the cut ends 4a, 4b with the groove 5. If so, portions of the cut ends 4a, 4b of about 5 mm in length are inserted into the groove 5 of 4 mm in depth. The cross-sectional area of the cut ends 4a, 4b inserted into the groove 5, that is, the cross-sectional area of the cut ends 4a, 4b squeezed by the retainers 22 becomes 15 mm² in case that the digestive tract 4 in the normal state is 1.5 mm in thickness, and becomes 20 mm² in case that the digestive tract 4 in the normal state is 2 mm in thickness, whereas the area of the circle of about 2 to 3 mm in diameter which is the intrinsic shape of the retainer 22 is too small as 3.14∼7.065 mm², so that the cut ends 4a, 4b of the digestive tract 4 is squeezed under the shrinking state by the retainers 22.

Then, as time goes by in the above state, the cut ends 4a, 4b of the digestive tract 4 adhere to each other outside the radially arranged retainers 22, as like as the apparatus 1 being used. When the parts of the anastomosed digestive tract 4 held by the retainers 22 inside the adhered portion are necrotized, the retainers 22 fall off inside the digestive tract 4 and then automatically excreted, so that a function and effect similar to that explained in the case of apparatus 1 can be obtained.

In addition to a shape memory alloy, the retainer 22 may be formed of a shape memory resin which has a similar property as the shape memory alloy or a spring member.

The retainer 22 can be mass produced easily by manufacturing a lengthy coil rounded many times and cutting it into several coil peaces rounded a predetermined times.

Further, in this embodiment, the retainer 22 of wire shape is used. However, a belt-like herical retainer 23 as shown in Fig. 14 may used instead of said retainer 22.

As described above, according to the apparatus of the present invention, since a plurality of retainers separately fall off inside a digestive tract after attaining their intended purpose and are each sufficiently small in size, the respective retainers are automatically excreted without the need of special attention, with the result that safety is improved, a burden imposed on patients is lessened, and the possibility of stenosis is reduced.

Where saccharides are used as the soluble material of the support tube, human bodies are less affected. Where the retainers are each formed in a circle, the retainers become suitable for mass production. Further, where the retainers are each made of a shape memory alloy or resin, the force of closing the open opposite ends of each retainer can be sustained until the time of use, even when a storage period prior to use is prolonged, and the retainers can easily buried into the support tube. Where the retainers use spring member, they can be manufactured less expensively. Where the retainers are each provided at its open opposite ends with magnets attracting to each other, holding portions of the cut ends of the digestive tract are brought into aurface-to-surface pressure contact.

## Claims

1. An apparatus for anastomosing a digestive tract comprising a support tube (2) formed of mainly a soluble material, an annular groove (5) being defined in the outer periphery of the support tube (2), and a plurality of retainers (3, 11) characterised in that each retainer (3,11) is endowed with a property of closing its open ends (3a, 3b), disposed around the groove (5) in the support tube (2) at a plurality of locations along both the inner side surfaces and the inner bottom surface of the groove (5) in a radial pattern as a whole, in a state that said both opposite ends (3a, 3b) are opened, the retainers (3, 11) being released from the support tube (2) with their open opposite ends (3a, 3b) closed as the soluble material of the support tube (2) is dissolved.

2. The apparatus for anastomosing a digestive tract according to claim 1 wherein said support tube (2) is made of saccharides.

3. The apparatus for anastomosing a digestive tract according to claim 1 or 2 wherein said ends (3a, 3b) of the retainer (3,11) are opposite to each other when they are closed.

4. The apparatus for anastomosing a digestive tract according to claim 1, 2 or 3 wherein said retainer (3, 11) is embedded in the support tube (2) along both the inner side surfaces and the inner bottom surface of the groove (5) in a substantially U-shaped state with both ends (3a, 3b) opened.

5. The apparatus for anastomosing a digestive tract according to claim 1 or 2 wherein said ends (3a, 3b) of the retainer (3,11) are overlapped with each other when they are closed.

6. The apparatus for anastomosing a digestive tract according to claim 1, 2, 3 or 5 wherein said retainer (3, 11) is embedded in the support tube (2) along both the inner side surfaces and the inner bottom surface of the groove (5) in an arcuate state with both ends (3a, 3b) opened, said retainer (3,11) forming a circle when said ends (3a, 3b) are closed.

7. The apparatus for anastomosing a digestive tract according to claim 1, 2, 3, 4, 5 or 6 wherein said retainer (3, 11) is made of a shape memory alloy having a property of closing the open opposite ends at a predetermined temperature.

8. The apparatus for anastomosing a digestive tract according to claim 1, 2, 3, 4, 5 or 6 wherein said retainer (3, 11) is made of resin having a property of closing the open opposite ends at a predetermined temperature.

9. The apparatus for anastomosing a digestive tract according to claim 1, 2, 3, 4, 5 or 6 wherein said retainer (3, 11) is made using a spring member.

10. The apparatus for anastomosing a digestive tract according to claim 1, 2, 3, 4 or 6 wherein said retainer (3, 11) is provided at the open opposite ends with magnets (13) attracting to each other.

## Patentansprüche

1. Vorrichtung zur Anastomose eines Verdauungstraktes mit einem Trägerrohr (2) aus einem im wesentlichen löslichen Stoff, einer Ringnut (5) längs des äußeren Umfangs des Trägerrohres (2) und mit mehreren Haltern (3, 11), dadurch gekennzeichnet, daß jeder Halter (3, 11) an seinen freien Enden (3a, 3b) verschließbar ist, die Halter längs der Nut (5) in dem Trägerrohr (2) an mehreren Stellen längs den inneren Seitenflächen und den inneren Bodenflächen der Nut (5) in einem insgesamt radialen Muster derart angeordnet sind, daß die gegenüberliegenden Enden (3a, 3b) geöffnet sind, und daß die Halter (3, 11) von dem Trägerrohr (2) mit ihren dann geschlossenen freien gegenüberliegenden Enden (3a, 3b) freigegeben werden, wenn der lösliche Stoff des Trägerrohres (2) aufgelöst ist.

2. Vorrichtung zur Anastomose eines Verdauungstraktes nach Anspruch 1, wobei das Trägerrohr aus Sacchariden besteht.

3. Vorrichtung zur Anastomose eines Verdauungstraktes nach Anspruch 1 oder 2, wobei die Enden (3a, 3b) der Halter (3, 11) einander gegenüberliegen, wenn sie geschlossen sind.

4. Vorrichtung zur Anastomose eines Verdauungstraktes nach Anspruch 1, 2 oder 3, wobei der Halter (3, 11) in dem Trägerrohr (2) längs der inneren Seitenflächen und der inneren Bodenfläche der Nut (5) in einer im wesentlichen U-Form mit beiden Enden (3a, 3b) geöffnet eingebettet ist.

5. Vorrichtung zur Anastomose eines Verdauungstraktes nach Anspruch 1 oder 2, wobei die Enden (3a, 3b) des Halters (3, 11) einander überlappen, wenn sie geschlossen sind.

6. Vorrichtung zur Anastomose eines Verdauungstraktes nach Anspruch 1, 2, 3 oder 5, wobei der Halter (3, 11) in dem Trägerrohr (2) längs der inneren Seitenflächen und der inneren Bodenfläche der Nut (5) in einer Bogenform mit beiden Enden (3a, 3b) geöffnet eingebettet ist und der Halter (3, 11) einen Kreis bildet, wenn die Enden (3a, 3b) geschlossen werden.

7. Vorrichtung zur Anastomose eines Verdauungstraktes nach Anspruch 1, 2, 3, 4, 5 oder 6, wobei der Halter (3, 11) aus einer Legierung mit Formerinnerungsvermögen mit der Eigenschaft besteht, daß die freien gegenüberliegenden Enden bei einer vorbestimmten Temperatur geschlossen werden.

8. Vorrichtung zur Anastomose eines Verdauungstraktes nach Anspruch 1, 2, 3, 4, 5 oder 6, wobei der Halter (3, 11) aus Plastik mit der Eigenschaft besteht, daß die freien gegenüberliegenden Enden bei einer vorbestimmten Temperatur geschlossen werden.

9. Vorrichtung zur Anastomose eines Verdauungstraktes nach Anspruch 1, 2, 3, 4, 5 oder 6, wobei der Halter (3, 11) unter Verwendung eines Federelementes hergestellt ist.

10. Vorrichtung zur Anastomose eines Verdauungstraktes nach Anspruch 1, 2, 3, 4 oder 6, wobei der Halter (3, 11) an seinen freien gegenüberliegenden Enden mit einander anziehenden Magneten (13) versehen ist.

## Revendications

1. Appareil pour l'anastomose d'un tube digestif, comprenant un tube de support (2) formé principalement d'une matière soluble, une rainure annulaire (5) étant définie dans la périphérie extérieure du tube de support (2) et un ensemble d'éléments de retenue (3, 11), caractérisé en ce que chaque élément de retenue (3, 11) est doué d'une propriété de fermer ses extrémités ouvertes (3a, 3b), et est disposé autour de la rainure (5) dans le tube de support (2) en un certain nombre d'emplacements le long des deux surfaces latérales intérieures et de la surface inférieure intérieure de la rainure (5) dans un schéma radial dans l'ensemble, dans un état où lesdites extrémités opposées (3a, 3b) sont ouvertes, les éléments de retenue (3, 11) étant dégagés du tube de support (2) avec leurs extrémités opposées ouvertes (3a, 3b) fermées lorsque la matière soluble du tube de support (2) est dissoute.

2. Appareil pour l'anastomose d'un tube digestif selon la revendication 1, dans lequel ledit tube de support (2) est formé de saccharides.

3. Appareil pour l'anastomose d'un tube digestif selon la revendication 1 ou 2, dans lequel lesdites extrémités (3a, 3b) de l'élément de retenue (3, 11) se font face l'une l'autre lorsqu'elles sont fermées.

4. Appareil pour l'anastomose d'un tube digestif selon la revendication 1, 2 ou 3, dans lequel ledit élément de retenue (3, 11) est encastré dans le tube de support (2) le long des deux surfaces latérales intérieures et de la surface inférieure intérieure de la rainure (5) dans un état globalement en forme de U avec les deux extrémités (3a, 3b) ouvertes.

5. Appareil pour l'anastomose d'un tube digestif selon la revendication 1 ou 2, dans lequel lesdites extrémités (3a, 3b) de l'élément de retenue (3, 11) se chevauchent l'une l'autre lorsqu'elles sont fermées.

6. Appareil pour l'anastomose d'un tube digestif selon la revendication 1, 2, 3 ou 5, dans lequel ledit élément de retenue (3, 11) est encastré dans le tube de support (2) le long des deux surfaces latérales intérieures et de la surface inférieure intérieure de la rainure (5) dans un état courbé avec les deux extrémités (3a, 3b) ouvertes, ledit élément de retenue (3, 11) formant un cercle lorsque lesdites extrémités (3a, 3b) sont fermées.

7. Appareil pour l'anastomose d'un tube digestif selon la revendication 1, 2, 3, 4, 5 ou 6, dans lequel ledit élément de retenue (3, 11) est fait d'un alliage à mémoire ayant la propriété de fermer les extrémités opposées ouvertes à une température prédéterminée.

8. Appareil pour l'anastomose d'un tube digestif selon la revendication 1, 2, 3, 4, 5 ou 6, dans lequel ledit élément de retenue (3, 11) est fait d'une résine ayant la propriété de fermer les extrémités opposées ouvertes à une température prédéterminée.

9. Appareil pour l'anastomose d'un tube digestif selon la revendication 1, 2, 3, 4, 5 ou 6, dans lequel ledit élément de retenue (3, 11) est fait en utilisant un élément élastique.

10. Appareil pour l'anastomose d'un tube digestif selon la revendication 1, 2, 3, 4, 5 ou 6, dans lequel ledit élément de retenue (3, 11) est muni aux extrémités opposées ouvertes d'aimants (13) s'attirant mutuellement.
